(19) Europäisches Patentamt

European Patent Office

Office européen des brevets

(11) Veröffentlichungsnummer : **0 428 554 B1**

(12) # EUROPÄISCHE PATENTSCHRIFT

(45) Veröffentlichungstag der Patentschrift :
26.05.93 Patentblatt 93/21

(51) Int. Cl.⁵ : **A61L 27/00, A61L 25/00**

(21) Anmeldenummer : 89908425.5

(22) Anmeldetag : 31.07.89

(86) Internationale Anmeldenummer :
**PCT/EP89/00893**

(87) Internationale Veröffentlichungsnummer :
**WO 90/01342 22.02.90 Gazette 90/05**

(54) **NEUE WERKSTOFFE FÜR DEN KNOCHENERSATZ UND KNOCHEN- BZW. PROTHESENVERBUND.**

Die Akte enthält technische Angaben, die nach dem Eingang der Anmeldung eingereicht wurden und die nicht in dieser Patentschrift enthalten sind.

(30) Priorität : **09.08.88 DE 3826915**

(43) Veröffentlichungstag der Anmeldung :
**29.05.91 Patentblatt 91/22**

(45) Bekanntmachung des Hinweises auf die Patenterteilung :
**26.05.93 Patentblatt 93/21**

(84) Benannte Vertragsstaaten :
**AT BE CH DE FR GB IT LI LU NL SE**

(56) Entgegenhaltungen :
**DE-A- 3 716 302**
**FR-A- 2 364 644**
**US-A- 4 645 503**

(73) Patentinhaber : **Henkel Kommanditgesellschaft auf Aktien Postfach 1100 Henkelstrasse 67 W-4000 Düsseldorf-Holthausen (DE)**

(72) Erfinder : **KRETSCHMANN, Josef Friedensstrasse 19 W-4018 Langenfeld (DE)**
Erfinder : **RITTER, Wolfgang Am Bandenfeld 74 W-5657 Haan (DE)**
Erfinder : **FUES, Johann-Friedrich Herzogstrasse 15 W-4048 Grevenbroich 5 (DE)**

(74) Vertreter : **von Kreisler, Alek, Dipl.-Chem. et al Patentanwälte Von Kreisler-Selting-Werner, Deichmannhaus am Hauptbahnhof W-5000 Köln 1 (DE)**

EP 0 428 554 B1

## Beschreibung

Die Erfindung betrifft ein verbessertes Kombinationsmaterial, das auf dem Gebiet der Werkstoffe für den Knochenersatz bzw. -verbund sowie bei der Einbindung von Prothesenmaterial in lebendes Knochengewebe mit Vorteil eingesetzt werden kann. Die Erfindung sieht dabei die Kombination bekannter körperverträglicher, keramischer Werkstoffe, insbesondere entsprechender bioaktiver Materialien auf Basis von Calciumphosphat, zusammen mit ausgewählten Oligoestern vor, die ebenfalls körperverträglich und vom menschlichen und tierischen Körper resorbierbar sind.

Diese Werkstoffe bilden im Körpermilieu mit dem Knochen einen Verbund und übernehmen temporär eine Stützfunktion. Sie werden im Zuge ihrer Resorption durch körpereigenes osteogenes Gewebe ersetzt, welches dann im Verlauf der natürlichen Knochenneubildung in kalzifiziertes und tragfähiges Knochengewebe umgewandelt wird. Solche Werkstoffe können in weiten Bereichen der orthopädischen Chirurgie und Kieferchirurgie überall dort eingesetzt werden, wo eine Regeneration unfall- bzw. krankheitsgeschädigter Bereiche des Skeletts anzustreben ist, z. B. unfallgeschädigte Bereiche von Röhrenknochen, nach Osteotomien u. dgl.

Bei der Suche nach einem geeigneten Knochenersatzwerkstoff für unbelastete bzw. vorwiegend auf Druck belastete Knochenabschnitte - beispielsweise bei der operativen Behandlung von Konturdegekten im Kiefer-Gesichtsbereich - haben in jüngerer Zeit Keramikwerkstoffe auf Basis von Calciumphosphatverbindungen Bedeutung gewonnen, vergl. hierzu beispielsweise Fischer-Brandies et al, "Knochenersatzwerkstoff Hydroxylapatit", Colloquium med. dent. "Der Zahnarzt" 30 (1986), 567 - 583.

Der praktische Einsatz solcher Knochenersatzwerkstoffe, die insbesondere häufig in gekörnter bzw. granulierter Form zur Verfügung stehen, bringt beträchtliche praktische Probleme. Es ist nicht nur ein Stoff zu finden, der über genügend Stabilität verfügt, der Knochenersatzwerkstoff soll dabei möglichst so formbar sein, daß die ursprüngliche Gestalt des Knochens wenigstens weitgehend wiederhergestellt werden kann. Im biologischen Sinne ist dabei zu fordern, daß das Implantat reaktionslos einheilt und auch bei längerer Verweilzeit keine pathologischen Gewebereaktionen auslöst. Form und Struktur eines solchen Implantats sollen solange erhalten bleiben, bis gegebenenfalls ein Ersatz durch körpereigenen Knochen stattfindet. Beim Einsatz im Kieferbereich muß das Material oft zusätzlich eine Prothesenbelastung erlauben.

Keramikwerkstoffe unterschiedlicher Resorptionsgeschwindigkeiten auf Basis von Calciumphosphaten und insbesondere Tricalciumphosphat "TCP" sowie das Pentacalcium-Hydroxid-Triphosphat "Hydroxylaptit" stehen heute - insbesondere auch in gesinterter Form - für den operativen Einsatz zur Verfügung. Die gesinterten Materialien zeichnen sich durch große Apatitkristal le und gegebenenfalls durch ein zusätzliches Verschmelzen der Einzelpartikel zu einem größeren Verbund aus. Die entsprechenden Materialien besitzen damit eine erhöhte Widerstandsfähigkeit im biologischen Milieu. In Abhängigkeit von dem jeweiligen Herstellungsverfahren und der angewendeten Sintertemperatur liegen Keramiken unterschiedlicher Porosität bzw. Dichte vor, wobei hier Mikroporen - etwa im Bereich von 1 μm bis zu etwa 100 μm - und größere Makroporen unterschieden werden können.

Der Einsatz eines solchen vergleichsweise feinteiligen bis gekörnten Materials, beispielsweise im Kieferbereich zur Restitution von Knochenabschnitten, die in ihrer Kontinuität erhalten sind, erfolgt nach den Angaben der eingangs zitierten Literaturstelle derart, daß das Zahnfleisch vom Knochen gelöst und eine Höhlung ausgebildet wird, die im erwünschten Ausmaß mit gekörntem und/oder granuliertem Ersatzwerkstoff auf Calciumphosphatbasis gefüllt wird. Die geöffnete Zahnfleischtasche wird dann vernäht und hält den Fullstoff an der gewünschten Stelle.

Mögliche Schwierigkeiten können in der gewünschten Formgebung bestehen. Das rieselfähige, keramische, feinpartikuläre Material zeigt keine eigene Formbeständigkeit, sondern paßt sich der vorgegebenen Form der ausgebildeten Gewebetasche an. Verschiebungen der Taschenfüllung zu einem späteren Zeitpunkt - bevor das Ersatzmaterial durch einwachsendes neues Knochengewebe hinreichend verfestigt ist - können auftreten. Andererseits kann es bei unfreiwilliger Öffnung der Wundnaht zum partiellen Verlust eingebrachter Füllkörper kommen.

Beträchtlich ältere Vorschläge - siehe hierzu die DE-OS 26 20 890 und DE-OS 26 20 891 - beschreiben partiell oder vollständig resorbierbare Knochenersatz-, Knochenverbund- oder Prothesenverankerungsmassen, die gesinterte Calciumphosphate entweder mit weichen plastischen organischen Materialien oder mit festen harten Polymerverbindungen kombinieren. Es sollen dadurch einerseits knetbare plastische Massen und andererseits harte Werkstoffe gebildet werden, die als Festkörper in den lebenden Organismus implantierbar sind. Die jeweils eingesetzte geschlossene organische Phase dieser Mischwerkstoffe kann aus bioresistenten oder resorbierbaren bzw. biodegradablen Polymeren bestehen. Als Beispiele für diese organische Phase sind genannt Polyester der Glycolsäure, der Milchsäure, Polyamide der Alpha-Aminosäuren, unmodifizierte oder modifizierte natürliche Polymere wie Gelatine oder Stärke, Triglyceride hoher Alkancarbonsäuren oder Ester von Polyhydroxiverbindungen sowie Polymethylmethacrylat und Polycyanacrylat. Insbesondere zur Herstel-

2

EP 0 428 554 B1

lung der entsprechenden harten Formkörper ist vorgesehen, den porösen keramischen Werkstoff mit dem polymerbildenden Monomeren zu tränken und dann die in situ Polymerisation der organischen Ausgangskomponenten in Gegenwart des keramischen Materials zu bewirken.

Die in diesen älteren Druckschriften dargestellten Modellvorstellungen haben sich bis heute für die ärztliche Praxis nicht ausgewirkt. Eine mögliche Erklärung liegt darin, daß der organischen Komponente dieser organisch/anorganischen Kombinationswerkstoffe im Reaktionsgeschehen des lebenden Organismus doch eine größere Bedeutung zukommt als seinerzeit angenommen wurde. Die Verträglichkeit solcher Implantate und ihre bestimmungsgemäße Funktion im lebenden Organismus wird in entscheidender Weise durch die Wahl und die Beschaffenheit der eingesetzten harzartigen oragnischen Phase mitbestimmt. Erst die sinnvolle konkrete Ausgestaltung gerade dieser organischen Polymerphasen sichert das angestrebte synergistische Zusammenwirken zwischen den drei Komponenten lebender Organismus, insbesondere Knochen/Phosphatkeramik/orgznische Polymerphase.

Von diesen Schwierigkeiten ausgehend schlägt die Erfindung vor, verbesserte Verbundwerkstoffe der geschilderten Art zu schaffen, die sowohl eine erleichterte Handhabung als auch ein rasches und sicheres Einwachsen des eingebrachten feinpartikulären Keramikwerkstoffes im unmittelbaren Kontakt mit intaktem Knochengewebe gewährleisten. Die Lösung der erfindungsgemäßen Aufgabe sieht vor, zusammen mit dem keramischen Werkstoff ausgewählte oligomere und/oder polymere Polyesterverbindungen einzusetzen, die körperverträglich und insbesondere auch im lebenden Organismus resorbierbar sind, den angestrebten Substitutionsprozeß am Knochen nicht durch unerwünschte Sekundärreaktionen stören, sondern in einer bevorzugten Ausführungsform sogar stimulieren und insbesondere auch befähigt sind, die formgebende Handhabung des Ersatzwerkstoffes bei der Operation zu erleichtern und die Formtreue des Implantats, insbesondere in der kritischen Übergangsphase bis zur hinreichenden körpereigenen Verfestigung zu verbessern.

Die Erfindung will dabei die Möglichkeit schaffen, in wiederholbarer Weise Kombinationsmaterialien der geschilderten Art in beliebigen Mengen stets gleichbleibender vorherbestimmbarer Qualität zur Verfügung zu stellen, wobei sich die Materialien ihrerseits durch optimale Gewebeverträglichkeit auszeichnen und auf Wegen hergestellt werden können, die den Ausschluß von körperfremden Hilfschemikalien ermöglichen.

Gegenstand der Erfindung sind dementsprechend wenigstens anteilsweise resorbierbare Knochenersatz- und/oder Knochenverbundwerkstoffe sowie Hilfsstoffe für die Einbindung von Prothesenmaterial in lebendes Knochengewebe, enthaliend als wesentliche Komponenten körperverträgliche Keramikwerkstoffe in Abmischung mit körperresorbierbaren Oligomeren und /oder Polymeren niederer Hydroxycarbonsäuren, insbesondere der Glycolsäure und/oder Milchsäure - im folgenden als "Harzkomponente" bezeichnet - wobei das Kennzeichen der Erfindung darin liegt, daß die Harzkomponente unter Mitverwendung von molekulargewichts-regelnden Coreaktanten aus der Klasse der 1- oder mehrfunktionellen Carbonsäuren bzw. enrsprechender Alkohole hergestellt und von freien Carboxylgrupnen praktisch befreit ist.

Erfindungswesentlich wird damit eine Kombination von Elementen für die Definition der Harzkomponente aus den Verbundmaterialen der geschilderten Art. Diese Harzkomponente ist bevorzugt aufgebaut auf polyesterartig verknüpften Glycolsäure und/oder Milchsäureeinheiten. Durch die Mitverwendung ausgewählter Coreaktanten wird jedoch - in an sich bekannter Weise - die Festlegung des mittleren Molekulargewichts ermöglicht, dem mitbestimmende Bedeutung für die Überlebensdauer der Harzkomponente im Stoffwechselgeschehen im lebenden Körper zukommt. Schließlich fordert die Erfindung den Einsatz von Harzkomponenten der geschilderten Art, die von freien Carboxylgruppen praktisch befreit sind. Hier findet sich ein wichtiges Element für die Körper- und insbesondere Gewebeverträglichkeit der erfindungsgemäß eingesetzten Harzkomponenten. Innerhalb der Klasse der molekulargewichtsbestimmenden Coreaktanten kommt in einer bevorzugten Ausführungsform der Erfindung dem Glycerin bzw. ausgewählten Glycerinpartialestern besondere Bedeutung zu, wie im nachfolgenden noch geschildert werden wird.

Zum Typ der körperverträglichen Oligomeren und/oder Polymeren von Hydroxycarbonsäuren sei verwiesen auf die DE-OS 32 29 540 (D 6652) und die zugehörige, noch nicht veröffentlichten deutschen Patentanmeldungen P 37 16 302.7 (D 7890) und P 38 25 211.2 (D 8293) der Anmelderin. Beschrieben werden in diesen Schutzrechten körperresorbierbare Wachse, die insbesondere zur mechanischen Blutstillung an körpereigenem Hartgewebe, bevorzugt am Knochen Einsatz finden sollen, und sich dadurch auszeichnen, daß sie aus bei Körpertemperatur zähviskosen bis Testen wachsartigen Polyester-Oligomeren bzw. -Polymeren von Hydroxycarbonsäuren bestehen. Geeignet sind für diesen Anwendungszweck insbesondere entsprechende Oligomere und /oder Polymere eines mittleren Molekulargewichtes im Bereich von etwa 200 bis 1500, vorzugsweise im Bereich von etwa 300 bis 1000. Die bevorzugten Polyesteroligomere sind aus Monohydroxycarbonsäuren gebildet, die vorzugsweise 2 bis 10 und insbesondere 2 bis 6 C-Atome aufweisen. Genannt sind im einzelnen Polyester-Oligomere aus Glycolsäure, Milchsäure, Hydroxypropionsäure, Hydroxybuttersäure und/oder Hydroxybenzoesäure. Die für die Praxis wichtigsten Hydroxycarbonsäuren zum Aufbau der hier betroffenen zähviskosen bis festen Polymerkomponenten sind die Glycolsäure und/oder die Milchsäure.

3

Es hat sich gezeigt, daß diese bisher im wesentlichen zur mechanischen Blutstillung am Knochen vorgeschlagenen Wachse und entsprechende Verbindungen auch noch höheren Molekulargewichts vorteilhafte Verwendung bei der Kombination mit mineralischen Knochenersatzstoffen, insbesondere der eingangs genannten Art, finden können. Werden insbesondere feste Oligomere und/oder Polymere der genannten Art in solchen Mengen mit dem partikulären, feinteiligen, anorganischen Knochenersatzwerkstoff vermischt, daß im Kombinationsmaterial die organische Phase als geschlossene Phase vorliegt, dann steht mit der Erfindung ein formbares und gewünschtenfalls formstabiles Material zur Verfügung, das in jeder beliebigen Raumform ausgebildet als Implantat mit körpereigenem Knochengewebe vereinigt werden kann. Die organische Polyesterphase ist nicht nur physiologisch unbedenklich, sie wird in der in den genannten älteren Schutzrechten der Anmelderin beschriebenen Weise resorbiert, ohne daß es dabei zur Bildung toxischer Abbauprodukte kommt. Zur gleichen Zeit - und in einer bevorzugten Ausführungsform weitgehend synchron damit - findet der Aufbau körpereigener Knochensubstanz und die Einbeziehung der abbauresistenten, anorganischen Partikel in das entstehende Neugewebe statt. Hier hat sich gezeigt, daß die Mitverwendung der erfindungsgemäßen ausgewählten Hydroxycarbonsäurepolyestermaterialien eine Neubildung von Knochengewebe gewünschter Struktur sogar ausdrücklich stimulieren kann. Es ist einleuchtend, daß durch den Einsatz des erfindungsgemäßen Verbundmaterials eine Verbesserung bei operativen Eingriffen mit Knochenersatzwerkstoffen auf Calciumphosphatbasis in mehrfacher Hinsicht erreicht werden kann.

Im einzelnen gelten zur organischen Polyesterphase der erfindungsgemäßen Verbundmaterialien die Angaben der genannten älteren Anmeldungen der Anmelderin, jedoch mit der Maßgabe, daß der Bereich des mittleren Molekulargewichts hier breiter gewählt sein kann. Er liegt üblicherweise im Bereich von etwa 200 bis 10000 und bevorzugt im Bereich von etwa 300 bis 5000 g/Mol.

Die bei Körpertemperatur bevorzugt zähviskosen bis festen Polyester-Oligomeren des angegebenen durchschnittlichen Molekulargewichtsbereiches enthalten endständige Coreaktanten zur Regulierung des Molekulargewichtes. Besonders wichtige Hydroxycarbonsäuren zur Ausbildung des Polyester-Oligomersegments sind die bereits genannte Glycolsäure, die isomeren Milchsäuren, die gegebenenfalls isomeren Alpha- oder Beta-Hydroxypropionsäuren, die gegebenenfalls isomeren Alpha-, Beta- oder Gamma-Hydroxybuttersäuren, o-Hydroxybenzoesäure (Salicylsäure), m-Hydroxybenzoesäure und/oder p-Hydroxybenzoesäure (Anissäure). Es können dabei bestimmte Isomere der genannten Säuren als auch Gemische davon zum Einsatz kommen. Durch Einsatz von Mischungen verschiedener Hydroxycarbonsäuren, insbesondere durch gemeinsame Verwendung von Glycolsäure und Milchsäure, läßt sich weitgehend Einfluß auf die Abbaugeschwindigkeit des Wachses durch körpereigene Abbaureaktionen nehmen.

Hochpolymere der hier geschilderten Art und ihr Einsatz auf dem medizinischen Sektor sind bekannt. Sie besitzen üblicherweise Fasereigenschaften. Ihre Verträglichkeit und Abbaubarkeit sind eingehend untersucht. Bekannt sind beispielsweise synthetische, im Organismus resorbierbare Fasermaterialien auf der Basis von Polyglycolsäure und Polymilchsäure, vergl. hierzu beispielsweise die US-PSen 3 297 033, 3 422, 871, 3 626 948, 2 668 162, 2 676 945 und 2 703 316. Weitere Literaturnachweise zum Einsatz solcher hochpolymeren Materialen finden sich in der genannten DE-OS-32 29 540.

Zur gezielten wiederholbaren Einstellung des Oligomerisierungsgrades und damit der gewünschten zähviskosen bis festen Konsistenz des Reaktionsproduktes werden bei seiner Herstellung einfunktionelle und /oder mehrfunktionel le Alkohole bzw. entsprechende Carbonsäuren als Coreaktanten mitverwendet. Geeignete Coreaktanten, und hier insbesondere Beispiele für die wichtige Klasse alkoholischer Coreaktanten, sind im einzelnen ebenfalls in den genannten älteren Anmeldungen beschrieben. Besondere Bedeutung kann in diesem Zusammenhang dem Glycerin zukommen, das zur Optimierung der Gewebeverträglichkeit der zähviskosen bis festen organischen Phase verwendet werden kann, so wie es im Rahmen der älteren Anmeldung P 37 16 302.7 (D 7890) beschrieben ist. Neben dem Glycerin kommt seinen Partialestern mit insbesondere $C_{12-18}$-Alkoholen Bedeutung zu.

Unerwünschte Gewebeschädigungen beim Einsatz solcher körperresorbierbarer Harze bzw. Wachse können besonders sicher dann vermieden werden, wenn durch die Herstellung des abbaubaren Wachses sichergestellt wird, daß dessen Gehalt an nicht-reagierten Carboxylgruppen zumindest stark abgesenkt oder besser noch praktisch vollständig beseitigt wird. Die statistische Molekulargewichts-Verteilung, die sich unmittelbar aus der Herstellung der Wachse ergibt, läßt noch immer einen gewissen Anteil an freien Carboxylgruppen im oligomeren Reaktionsgemisch zurück und zwar auch dann, wenn an sich der gewünschte mittlere Zahlenwert für das Molekulargewicht eingestellt ist. Dabei kann es sich hier auch noch um Anteile an freien monomeren Hydroxycarbonsäuren handeln.

Zur Senkung des Gehalts der organischen Phase an freien Carboxylgruppen stehen verschiedene Möglichkeiren zur Verfügung. In der älteren Anmeldung P 37 16 302.7 (D 7890) wird in einer bevorzugten Ausführungsform das wachsartige Material von seinem Gehalt an nicht umgesetzten Ausgangskomponenten so weitgehend gereinigt, daß der Gehalt an nicht-reagierten Hydroxycarbonsäuren auf Restgehalte unterhalb 0,5 Gew. -% und vorzugsweise auf Restgehalte unter etwa 0,2 Gew.-% abgesenkt ist. In der Regel können Restgehalte an nicht-reagierten Hydroxycarbonsäuren in den zum Einsatz kommenden Wachsen bei oder unterhalb von 0,1 Gew.-% liegen. Die Reinigung des primär anfallenden Oligomeren von nicht umgesetzten Komponenten bzw. Umsetzungsprodukten eines unerwünschten niederen Molgewichtes kann in einfacher Weise wie folgt bewerkstelligt werden: Man vermischt das primär angefallene Edukt mit einem wassermischbaren organischen Lösungsmittel, beispielsweise mit Aceton, Methanol oder Ethanol oder dergleichen und gibt die dabei angefallene Ausschlämmung anschließend in ein Lösungsmittel, das die erwünschten Oligomerfraktionen nicht löst, jedoch ein hinreichendes Lösungsmittel für nicht umgesetzte Komponenten bzw. Reaktionsprodukte niederen Molekulargewichtes ist. Als besonders geeignetes Fällungsmedium für diese zweite Verfahrensstufe hat sich Isopropanol erwiesen. So sieht ein zweckmäßiges Reinigungsverfahren vor, primär angefallenes Oligomerisationsprodukt und Lösungsmittel von der Art des Acetons, Methanols und dergleichen etwa im Verhältnis von 1 : 1 zu mischen und intensiv zu digerieren. Die angefallene Feststoffsuspension wird äann in das mehrfache Volumen, beispielsweise das 7 bis 12-fache an Isopropanol eingegeben und abfiltriert. Die Flüssigphase wird abgesaugt, zweckmäßig mit Isopropanol nachgewaschen und getrocknet. Die Einzelheiten dieser Reinigungsbehandlung sind naturgemäß nur beispielhaft zu verstehen, sie können im Rahmen des Fachwissens variiert werden.

Erfindungsgemäß wird darüberhinausgehend in einer ganz anderen Weise das primär anfallende Oligomerisationsprodukt von freien Carboxylgruppen befreit. In dieser Ausführungsform wird eine Neutralisation der freien Carboxylgruppen mit geeigneten salzbildenden und körperverträglichen Kationen vorgenommen. Zu nennen sind hier in erster Linie Alkali- und/oder die Erdalkalimetalle und insbesondere Calcium und Magnesium, gewünschtenfalls aber auch Aluminium. Einzelheiten hierzu finden sich in der genannten älteren Anmeldung P 38 25 211.2 (D 8293) der Anmelderin.

Zur Natur geeigneter keramischer Werkstoffmaterialien auf Basis Calciumphosphat und zu ihrer chemischen Definition sei auf die eingangs zitierte Veröffentlichung von Fischer-Brandies et al verwiesen. Bevorzugtes keramisches Material ist hinreichend gereinigter Hydroxylapatit und/oder Tricalciumphosphat. Im Austausch können andere Ionen in das Kristallgitter aufgenommen werden, wie es in der zitierten Literaturstelle angegeben ist. Gesintertes und gewünschtenfalls mikro- und/oder makroporöses Material bzw. Mischungen solcher Materialien können zum Einsatz kommen.

In einer bevorzugten Ausführungsform enthält das neue Verbundmaterial pulverförmige und/oder granulierte keramische Massen auf Calciumphosphatbasis in inniger Abmischung mit der organischen Phase, wobei die anorganische, feinteilige Materialphase als disperse Füllstoffphase in Hydroxycarbonsäure-Oligomeren bzw. -Polymeren vorliegt, die als kontinuierliche bzw. geschlossene Phase den dispers verteilten Füllstoff umhüllt. In einer weiteren Ausführungsform eignen sich aber auch Kombinationsmaterialien aus den hier genannten anorganischen und organischen Bestandteilen, bei denen die organische Phase in derart begrenzter Menge eingesetzt wird, daß ein Granulat- bzw. Feinkorncharakter des Kombinationsmaterials grundsätzlich erhalten ist, wobei jedoch Kerne aus anorganischem Material der geschilderten Art von dem organischen Werkstoffbestandteil umhüllt und - im Falle poröser anorganischer Werkstoffmaterialien - gegebenenfalls auch wenigstens anteilsweise durchdrungen sind.

Die hier zuletzt geschilderte erste Ausführungsform mit der Harzkomoponente als kontinuierlicher Stoffphase, die dispers verteilte Keramikwerkstoffpartikel enthält, kann als knetbare Masse ausgebildet sein, wobei die Knetbarkeit gewünschtenfalls durch Einsatz mäßig erhöhter Temperaturen geschaffen wird, bei Raumtemperatur und bevorzugt auch bei Körpertemperatur gleichwohl eine formstabile feste Masse vorliegt. Diese Ausführungsform umfaßt aber auch Materialien, die zur formgebenden Verarbeitung spanabhebend bzw. durch Schneiden, Fräsen u. dgl. zu verarbeiten sind. Die zweite der zuletzt geschilderten Ausführungsformen sieht ein disperses Knochenaustauschmaterial vor, das die Harzkomponente wenigstens als Umhüllung der Einzelteilchen, gewünschtenfalls aber auch aufgesogen in das Innere der Einzelteilchen aufweist. Hier steht für den Einsatz der Harzkomponente nicht die Ausbildung vorgegebener Raumformen im Vordergrund, entscheidend ist der Verschluß und/oder die Auffüllung der porigen Struktur des anorganischen Materials. Damit wird einerseits der Einschluß unerwünschter Sekret- und/oder Blutreste im Wundbereich verhindert, zum anderen tritt auch in dieser Form die das Knochenneuwachstum stimulierende Funktion der Harzkomponente in Erscheinung.

Liegen die neuen Kombinationswerkstoffe als kohäsive gebenenfalls in der Wärme formbare Massen vor, so kann als Keramikwerkstoff ausschließlich pulverförmiges Calciumphosphat vorgesehen sein, wobei als Obergrenze Tür den Teilchenbereich der pulverförmigen Materialien 100 bis 200 μm anzusehen sind. Knochenartige feste Ersatzmaterialien dieser Art fallen insbesondere dann an, wenn der Gehalt dieser Werkstoffe an keramischen Pulver bei wenigstens etwa 30 Gew. -% und bevorzuat im Bereich von 40 bis 80 Gew. -%, insbesondere 40 bis 65 Gew. -% liegt.

Enthalten andererseits die erfindungsgemäßen Kombinationswerkstoffe die Keramikmaterialien in Granulatform dann gelten die folgenden Zahlenangaben für die bevorzugten Teilchengrößen der Granulate und die jeweils eingesetzten Mischungsverhältnisse: Bevorzugter Teilchendurchmesser der Granulate im Bereich von 0,1 bis 3 mm, insbesondere von 0,1 bis 2 mm und bevorzugt 0,2 bis 1 mm. Bevorzugter Granulatgehalt im Bereich von 20 bis 70 Gew.-% und insbesondere im Bereich von 20 bis 60 Gew.-%, wobei dem Bereich von 25 bis 50 Gew.-% besondere Bedeutung zukommen kann. Alle die hier genannten Gewichtsprozentangaben beziehen sich jeweils auf das Gesamtgewicht des erfindungsgemäßen Kombinationsmaterials.

In einer besonderen Ausführungsform der Erfindung liegen die keramischen Werkstoffe in wenigstens bimodaler Teilchengrößenverteilung vor, wobei hier insbesondere Gemische von Pulver und Granulat eingesetzt werden können. Allerdings sind auch andere Kombinationen, beispielsweise der Einsatz eines Gemisches urterschiedlich großer Granulatteilchen möglich. Durch diese Maßnahmen können vorbestimmte Eigenschafts- bzw. Wirkungskombinationen bei der Verarbeitung und/oder beim Verweilen des Verbundmaterials im Körper eingestellt werden. So ist beispielsweise hierdurch die Regelung eines "offenen Porendurchmessers" für das gesamte Materialstück möglich, d. h. es gelingt damit die Regelung der durchschnittlichen Gangdurchmesser, die mit der Harzkomponente ausgefüllt die Keramikwerkstoffe voneinander trennen bzw. miteinander verbinden. Auf diese Weise kann eine Optimierung des Freiraumes für das primär einsetzende frische Knochenwachsium gefunden werden, das dann in nachfolgenden Phasen des Heilungsprozesses zur Resorption und Aufnahme auch des keramischen Anteils in den lebenden Körper führt.

Liegen wenigstens bimodale Größenverteilungen bezüglich der keramischen Werkstoffe vor, so können Gemische von Pulver und Granulat bevorzuat sein, die Pulveranteile von 5 bis 30 Gew.-% und Granulatanteile von 15 bis 50 Gew.-% - auch hier Gewichtsprozent jeweils bezogen auf Gesamtwerkstoff - aufweisen.

In einer besonders bevorzugten Ausführungsform der Erfindung werden Harzkomponenten auf Basis der niederen Hydroxycarbonsäuren und insbesondere der Glycolsäure und/oder der Milchsäure eingesetzt, die durch Kondensation monomerer Ausgangselemente in Abwesenheit von Katalysatoren hergestellt worden sind. Eine solche Eigenkondensation der Glycolsäure und der Milchsäure gelingt bekanntlich unter dem Einfluß der die Veresterung katalysierenden freien Carbonsäuregruppen des Einsatzgutes. Auch hier wird in Gegenwart der das gewünschte Molekulargewicht regelnden Coreaktanten, insbesondere in Gegenwart von Glycerin gearbeitet. Ganz besonders in diesen Ausführungsformen ist die abschließende Beseitigung freier Carboxylgruppen im Kondensationsprodukt, insbesondere durch Salzbildung mit beispielsweise Calcium und/oder Magnesium wichtig. Werden solche Harzkomponenten mit den Keramikwerkstoffen gemischt, wird in optimaler Weise der Eintrag unerwünschter Fremdelemente in den Kombinationswerkstoff und letztlich damit in den lenenden Körper ausgeschlossen. Die Polykondensation der Harzkomponente kann in diesem Fall in an sich bekannter Weise zunächst unter Normaldruck und Abtrennung des anfallenden Reaktionswassers und gewünschtenfalls in späten Stadien der Polykondensation unter zunehmenden Vakuum durchgeführt werden.

Die Vermischung der Harzkomponenten mit den pulverförmigen und/oder -granulierten Keramikwerkstoffen kann in an sich bekannter Weise erfolgen. Bevorzugt wird dabei mit der Schmelze der Harzkomponenten gearbeitet. Insbesondere bei der Einarbeitung beschränkter Mengen der Harzkomponente in vergleichsweise größere Anteile der Keramikwerkstoffe kann es zweckmäßig sein, zum Vermischen inerte Hilfslösungsmittel mitzuverwenden, die nach dem Vermischungsvorgang mit abgetrennt werden. Bevorzugt sind hier hinreichend leichtflüchtige Lösungsmittel von der Art des Acetons, leichtflüchtiger Alkohole, Ester u. dgl.

**Beispiele**

1. Herstellung und Beschreibung der resorbierbaren Harzkomponenten

a) Harze, hergestellt aus Glycolsäure/Glycerin

Allgemeine Vorschrift zur Herstellung der Umsetzungsprodukte von Glycolsäure mit Glycerin

In einem Dreihalskolben mit Rührer und Destillationsbrücke werden Glycolsäure und Glycerin vorgelegt. Unter vollständiger Inertisierung mit Stickstoff wird auf 150 °C aufgeheizt und die Reaktion innerhalb von 3 bis 5 Stunden so lange weitergeführt, bis sich kein Reaktionswasser mehr abspaltet. Dann wird bei 150 °C vorsichtig auf 10 Torr evakuiert. Nach weiteren 2 Stunden bei diesen Reaktionsbedingungen wird auf 100 ° abgekühlt, das Vakuum aufgehoben und das Produkt wie nachfolgend unter 2. beschrieben neutralisiert und noch heiß abgefüllt.

Die Zusammensetzung der Ansätze und die Oligomereigenschaften sind der Tabelle 1 zu entnehmen.

Tabelle 1

Oligohydroxycarbonsäuren aus Glycolsäure und Glycerin

| Beispiel | Edukte Glycolsäure mol | Glycerin mol | Ausbeute Reaktions- wasser % | Viskosität bei Meßtemperatur | Beschaffenheit |
|---|---|---|---|---|---|
| 1 | 8 | 1 | 100 | 2450 mPas/65-70$^{o}$C | hochviskos, leicht gelb |
| 2 | 9 | 1 | 99,1 | 3950 mPas/65-70$^{o}$C | weich, pasten- artig, gelblich |
| 3 | 12 | 1 | 99,0 | – | hart, weiß |

EP 0 428 554 B1

b) Harze, hergestellt aus Lactid und Glycerin

Allgemeine Vorschrift zur Herstellung der Umsetzungsprodukte von Lactid und Glycerin

Lactid (L(-)-Lactid N, der Firma Böhringer Ingelheim) und Glycerin wurden in einer üblichen Laborapparatur unter Stickstoff und unter Rühren innerhalb von einer Stunde auf 195 °C erwärmt. Man ließ dann 3 Stunden bei 195 °C reagieren und füllte nach Neutralisation wie unter 2. beschrieben heiß ab. Als Katalysator war eine Sn-II-Chlorid-Lösung in Ether zugesetzt (7 ml einer Lösung von 2,5 g $SnCl_2$ in 1000 ml Ether bei der Umsetzung von 3 mol Lactid mit einem mol Glycerin).

Die Zusammensetzung der Ansätze und die Oligomereigenschaften sind der Tabelle 2 zu entnehmen.

## Tabelle 2

Oligohydroxycarbonsäuren aus Glycerin und Lactid

| Beispiel | Edukte | | Beschaffenheit | Viskosität bei 65-70°C | Gehalt an freier Milchsäure (Gew.-%) |
| --- | --- | --- | --- | --- | --- |
| | Glycerin mol | Lactid mol | | | |
| 4 | 1 | 5 | weich, klar | 2500 mPas | 0,125 % |
| 5 | 1 | 8 | fest, spröde, klar | 6000 mPas | - |

2. Verfahren zur Befüllung der Harze und Eigenschaften der Mischungen

Von den unter 1. beschriebenen Harzen wurde durch Titration der Gehalt an freier Säure (Glycolsäure/Milchsäure) bestimmt. Die freie Säure wurde durch Zugabe der äquimolaren Menge $CaCO_3$ und intensiver Durchmischung neutralisiert.

Die Schmelze der neutralisierten Harze wurde unter Rühren bei Temperaturen von 120 °C (niedrige Befullung) bis 160 °C (hohe Befüllung) mit Hydroxylapatit versetzt und für 15 Min. intensiv durchmischt. Die Befüllung der Harze mit über 60 % Hydroxylapatit erwies sich als schwierig. Hierzu wurde das Harz in Aceton (1 : 1) gelöst, mit Hydroxylapatit unter Rühren befüllt und anschließend das Lösungsmittel abgedampft.

Nach dem Erkalten (Lagerung für 24 Stunden) in einer Aluminiumschale) wurde die Konsistenz beurteilt und die Shore-Härte gemessen.

Die Ergebnisse sind in den Tabellen 3 bis 7 aufgeführt.

Tabelle 3

Eigenschaften der befüllten Systeme

| Resorbierbares Harz Nr. 1 (Glycolsäure/ Glycerin 8 : 1) | Befüllungsgrad /Gew.-% | Füllstoff und Eigenschaften gesintertes Hydroxylapatit-Granulat Korngröße 0,25 – 0,5 mm Konsistenz | Shore-A-Härte |
|---|---|---|---|
| a | 10 | zweiphasiges Gemisch aus Wachs und sedimentiertem Apatit ohne Kohäsion | – |
| b | 20 | | – |
| c | 30 | | – |
| d | 40 | homogen befüllte, flexible Masse | 46 |
| e | 50. | | 47 |
| f | 60 | | 64 |
| g | 70 | mit Harz benetztes Granulat | – |
| | | gesintertes Hydroxylapatit-Granulat Korngröße 0,50 – 1,0 mm | |
| h | 50 | homogen befüllte, flexible Masse | 75 |
| i | 60 | | 68 |
| j | 70 | | 81 |
| k | 80 | mit Harz benetztes Granulat | – |

EP 0 428 554 B1

Tabelle 4

Eigenschaften der befüllten Systeme

| Resorbierbares Harz Nr. 2 (Glycolsäure/ Glycerin 9 : 1) | Befüllungsgrad /Gew.-% | Füllstoff und Eigenschaften Hydroxylapatit Pulver Konsistenz | Shore-A-Härte |
|---|---|---|---|
| a | 10 | wachsartig verformbar | 37 |
| b | 20 | wachsartig verformbar | 46 |
| c | 30 | wachsartig | 76 |
| d | 40 | wachsartig | 78 |
| e | 70 | leicht brüchiger Feststoff ohne Kohäsion | 5 |
| | | gesintertes Hydroxylapatit-Granulat Korngröße 0,25 - 0,5 mm | |
| f | 40 | zweiphasiges Gemisch (sedimentiertes Apatit) | – |
| g | 50 | homogen befüllte, harte Masse | 53 |
| h | 55 | homogen befüllte, harte Masse | 67 |
| i | 60 | homogen befüllte, harte Masse | 57 |
| | | gesintertes Hydroxylapatit-Granulat Korngröße 0,50 - 1,0 mm | |
| ·j | 50 | homogen befüllte, harte Masse | 64 |

EP 0 428 554 B1

## Tabelle 5

Eigenschaften der befüllten Systeme

| Resorbierbares Harz Nr. 3 (Glycolsäure/ Glycerin 12 : 1) | Befüllungsgrad /Gew.-% | Füllstoff und Eigenschaften gesintertes Hydroxylapatit-Granulat Korngröße 0,25 - 0,5 mm Konsistenz | Shore-A-Härte |
|---|---|---|---|
| a | 40 | zweiphasiges Gemisch aus Harz und sedimentiertem Apatit | – |
| b | 50 | } homogen befüllte, harte Masse | 57 |
| c | 60 | | 54 |
| | | gesintertes Hydroxylapatit-Granulat Korngröße 0,50 - 1,0 mm | |
| d | 50 | homogen befüllte, harte Masse | 71 |

EP 0 428 554 B1

## Tabelle 6

Eigenschaften der befüllten Systeme

| Resorbierbares Harz Nr. 4 (Lactid/ Glycerin 5 : 1) | Befüllungsgrad /Gew.-% | Füllstoff und Eigenschaften Hydroxylapatit Pulver Konsistenz | Shore-A-Härte |
|---|---|---|---|
| a | 10 | wachsartig verformbar | 27 |
| b | 20 | wachsartig verformbar | 38 |
| c | 30 | wachsartig verformbar | 65 |
| d | 40 | wachsartig | 72 |
| e | 50 | wachsartig | 78 |
| f | 70 | leicht brüchiger Feststoff ohne Kohäsion | 12 |

Tabelle 7

Eigenschaften der befüllten Systeme

| Resorbierbares Harz Nr. 5 (Lactid/ Glycerin 8 : 1) | Befüllungsgrad / Gew.-% | Füllstoff und Eigenschaften gesintertes Hydroxylapatit-Granulat Korngröße 0,25 – 0,5 mm Konsistenz | Shore-A-Härte |
|---|---|---|---|
| a | 40 | ⎫ | 70 |
| b | 50 | ⎬ homogen befüllte, harte Masse | 70 |
| c | 60 | ⎭ | 74 |
| | | gesintertes Hydroxylapatit-Granulat Korngröße 0,5 – 1,0 mm | |
| d | 50 | homogene befüllte, harte Masse | 74 |

Meßmethoden/Verwendete Zuschlagstoffe

1) Die Shore-A-Härte wurde mit dem Gerät der Fa. Zwick (nach DIN 53505, ASTM D 2240) durch die Dorneindringtiefe bestimmt.

2) Hydroxylapatit Pulver

penta-Calciumhydroxy-triphosphat, $Ca_5(PO_4)_3(OH)$

Fa. Merck/Darmstadt

Hydroxylapatit gesintertes Granulat

$Ca_5(PO_4)_3(OH)$

Fa. Heyl; Chem.-pharm. Fabrik/Berlin 37

3) Die Bestimmung der Viskosität urde mit dem nachfolgenden Gerät durchgeführt:

Epprecht Torsionsviskosimeter Typ TVB. Meßkörper 4 bei 200 UpM/70 °C (temperierte Proben).

## Patentansprüche

1. Wenigstens anteilsweise resorbierbare Knochenersatz- und/oder Knochenverbundwerkstoffe sowie Hilfsstoffe für die Einbindung von Prothesenmaterial in lebendes Knochengewebe enthaltend als wesentliche Komponenten körperverträgliche Keramikwerkstoffe in Abmischung mit körperresorbierbaren Oligomeren und/oder Polymeren niederer Hydroxycarbonsäuren, insbesondere der Glycolsäure und/oder Milchsäure (Harzkomponente), dadurch gekennzeichnet, daß die Harzkomponente unter Mitverwendung von molekulargewichts-regelnden Coreaktanten aus der Klasse 1- oder mehrfunktioneller Carbonsäuren bzw. entsprechender Alkohole hergestellt und von freien Carboxylgruppen praktisch befreit ist.

2. Werkstoffe nach Anspruch 1, dadurch gekennzeichnet, daß die Harzkomponente unter Mitverwendung von Glycerin und/oder Glycerinpartialestern mit insbesondere höheren Monocarbonsäuren - bevorzugter C-Zahlbereich $C_{12-18}$ - hergestellt worden ist.

3. Werkstoffe nach Anspruch 2, dadurch gekennzeichnet, daß in der Harzkomponente ursprünglich vorliegende freie Carboxylgruppen zu körperverträglichen Salzgruppen, insbesondere solchen der Alkali- und/oder Erdalkalimetalle bzw. des Aluminiums umgewandelt sind, wobei entsprechende Calciumsalzgruppen besonders bevorzugt sein können.

4. Werkstoffe nach Ansprüchen 1 bis 3, dadurch gekennzeichnet, daß sie wenigstens anteilsweise, bevorzugt überwiegend oder gar ausschließlich bioaktive Keramikwerkstoffe, insbesondere aus der Klasse resorbierbarer und/oder nicht resorbierbarer Calciumphosphatkeramiken in Pulver- und/oder in Granulatform enthalten.

5. Werkstoffe nach Ansprüchen 1 bis 4, dadurch gekennzeichnet, daß sie gekörnte Keramikwerkstoffe enthalten, die insbesondere auch als poröses Granulat vorliegen können, das mit der Harzkomponente wenigstens anteilsweise gefüllt und/oder umhüllt ist.

6. Werkstoffe nach Ansprüchen 1 bis 5, dadurch gekennzeichnet, daß sie als bioaktive Keramikwerkstoffe Tricalciumphosphat und/oder Hydroxylapatit in ungesinterter und/oder gesinterter Form enthalten.

7. Werkstoffe nach Ansprüchen 1 bis 6, dadurch gekennzeichnet, daß sie als kohäsiver, bei Raumtemperatur und vorzugsweise auch bei Körpertemperatur knetbarer oder starrer Verbundwerkstoff ausgebildet sind, der die Harzkomponente als geschlossene Phase enthält, in der die keramischen Werkstoffe in bevorzugt homogener Verteilung dispergiert vorliegen.

8. Werkstoffe nach Ansprüchen 1 bis 6, dadurch gekennzeichnet, daß sie als disperses Verbundmaterial ausgebildet sind, dessen Einzelteilchen durch Keramikpartikel bzw-. Partikelagglomerate gebildet werden, die mit der Harzkomponente umhüllt und gegebenenfalls wenigstens anteilsweise getränkt sind.

9. Werkstoffe nach Ansprüchen 1 bis 8, dadurch gekennzeichnet, daß sie Harzkomponenten eines mittleren Molekulargewichts im Bereich von 200 bis 10000 g/Mol und insbesondere von 300 bis 5000 g/Mol enthalten.

10. Werkstoffe nach Ansprüchen 1 bis 9, dadurch gekennzeichnet, daß sie als kohäsive, in der Wärme formbare Masse vorliegen und die Keramikwerkstoffe ausschließlich in Pulverform bei Gehalten dieser Pulver oberhalb etwa 30 Gew.-%, bevorzugt im Bereich von 40 bis 65 Gew.-% enthalten, wobei die bevorzugten Teilchengrößen der Pulver unterhalb 100 μm liegen.

11. Werkstoffe nach Ansprüchen 1 bis 9, dadurch gekennzeichnet, daß sie als kohärente Masse die Keramikwerkstoffe in Granulatform - bevorzugter Teilchendurchmesser der Granulate von 0,1 bis 2 mm, insbesondere 0,2 bis 1 mm - enthalten, wobei der Granulatgehalt bevorzugt im Bereich von 20 bis 60 Gew.-% und insbesondere im Bereich von 25 bis 50 Gew.-% liegt.

12. Werkstoffe nach Ansprüchen 1 bis 11, dadurch gekennzeichnet, daß die keramischen Werkstoffe in wenigstens bimodaler Größenverteilung, insbesondere als Gemisch von Pulver und Granulat vorliegen, wobei bevorzugt der Pulveranteil 5 bis 30 Gew.-% und der Granulatanteil 15 bis 50 Gew.-% - Gewichtsprozent jeweils bezogen auf Gesamtwerkstoff - ausmachen.

13. Werkstoffe nach Ansprüchen 1 bis 12, dadurch gekennzeichnet, daß als Harzkomponente Polymere der Glycolsäure und/oder der Milchsäure vorliegen, die keine Reste fremder Katalysatoren von der Herstellung enthalten.

## Claims

1. At least partly resorbable materials for replacing and/or joining bones and auxiliaries for implanting prosthesis material in living bone tissue, containing as essential components body-compatible ceramic materials in admixture with body-resorbable oligomers and/or polymers of lower hydroxycarboxylic acids, more particularly glycolic acid and/or lactic acid (resin component), characterized in that the resin component is produced using molecular-weight-regulating co-reactants from the class of mono- or polyfunctional carboxylic acids or corresponding alcohols and is substantially freed from free carboxyl groups.

2. Materials as claimed in claim 1, characterized in that the resin component has been produced using glycerol and/or glycerol partial esters with, in particular, higher monocarboxylic acids preferably containing from 12 to 18 carbon atoms.

3. Materials as claimed in claim 2, characterized in that free carboxyl groups originally present in the resin component are converted into body-compatible salt groups, more particularly those of the alkali metals and/or alkaline earth metals or aluminium, corresponding calcium salt groups being particularly preferred.

4. Materials as claimed in claims 1 to 3, characterized in that they contain at least partly, preferably predominantly or even exclusively bioactive ceramic materials, more particularly from the class of resorbable and/or non-resorbable calcium phosphate ceramics in powder and/or granular form.

5. Materials as claimed in claims 1 to 4, characterized in that they contain granulated ceramic materials, more particularly in the form of porous granules which may be at least partly filled and/or coated with the resin component.

6. Materials as claimed in claims 1 to 5, characterized in that they contain tricalcium phosphate and/or hydroxyl apatite in unsintered and/or sintered form as the bioactive ceramic materials.

7. Materials as claimed in claims 1 to 6, characterized in that they are in the form of cohesive composite materials which are kneadable or solid at room temperature and preferably at body temperature and which contain the resin component as continuous phase in which the ceramic materials are present in preferably homogeneous dispersion.

8. Materials as claimed in claims 1 to 6, characterized in that they are in the form of a disperse composite material of which the individual particles are formed by certamic particles or agglomerates which are coated and, optionally, at least partly impregnated with the resin component.

9. Materials as claimed in claims 1 to 8, characterized in that they contain resin components having an average molecular weight in the range from 200 to 10,000 g/mol and more particularly in the range from 300 to 5,000 g/mol.

10. Materials as claimed in claims 1 to 9, characterized in that they are present in the form of a cohesive, heat-formable compound and contain the ceramic materials solely in powder form, the powder contents being above about 30% by weight and preferably in the range from 40 to 65% by weight and the particle sizes of the powders preferably being below 100 μm.

11. Materials as claimed in claims 1 to 9, characterized in that, as a coherent compound, they contain the ceramic materials in the form of granules preferably having particle diameters of from 0.1 to 2 mm and more preferably from 0.2 to 1 mm, the content of granules preferably being in the range from 20 to 60% by weight and more preferably in the range from 25 to 50% by weight.

12. Materials as claimed in claims 1 to 11, characterized in that the ceramic materials are present in an at least bimodal particle size distribution, more particularly in the form of a mixture of powder and granules in which the powder makes up 5 to 30% by weight while the granules make up 15 to 50% by weight, based on the material as a whole.

13. Materials as claimed in claims 1 to 12, characterized in that polymers of glycolic acid and/or lactic acid containing no residues of foreign catalysts from their production are used as the resin component.

**Revendications**

1. Matériaux de remplacement d'os et/ou de liaison d'os pouvant être au moins en partie résorbés, ainsi que substances auxiliaires pour l'intégration de matériau de prothèse dans du tissu osseux vivant, contenant comme composants essentiels des matières de céramique tolérables pour le corps mélangées à des oligomères et/ou polymères d'acides hydroxycarboxyliques inférieurs, en particulier de l'acide glycolique et/ou de l'acide lactique (composant de la résine), matériaux caractérisés en ce que le composant de la résine est obtenu en utilisant conjointement des coréactifs régulateurs du poids moléculaire provenant d'acides carboxyliques de la classe 1 ou à plusieurs fonctions ou d'alcools correspondants et est pratiquement libéré de groupes carboxyle libres.

2. Matériaux selon la revendication 1, caractérisés en ce que le composant de la résine a été produit en utilisant conjointement du glycérol et/ou des esters partiels du glycérol avec en particulier des acides monocarboxyliques supérieurs, de préférence dans la gamme des indices de carbone $C_{12-18}$.

3. Matériaux selon la revendication 2, caractérisés en ce que dans le composant de la résine des groupes carboxyle existants à l'origine libres sont transformés en groupes de sels tolérables par le corps, en particulier de sels de métaux alcalins et/ou alcalino-terreux ou de l'aluminium, parmi lesquels on peut placer comme préférence particulière des groupes de sels de calcium adéquats.

4. Matériaux selon les revendications 1 à 3, caractérisés en ce qu'ils contiennent sous forme pulvérulente ou de granulés, des matières de céramique, au moins partiellement, préférentiellement de façon dominante ou même exclusivement bioactives, provenant particulièrement de la classe des céramiques phosphocalciques, pouvant être résorbées ou non résorbées.

5. Matériaux selon les revendications 1 à 4, caractérisés en ce qu'ils contiennent des matières céramiques granulées, qui peuvent aussi en particulier se présenter sous la forme de granulés poreux, qui sont au moins partiellement remplis du composant de la résine et/ou enveloppés par lui.

6. Matériaux selon les revendications 1 à 5, caractérisés en ce qu'ils contiennent comme matières céramiques biactives du phosphate tricalcique et/ou de l'apatite hydroxylique sous forme frittée et/ou non frittée.

7. Matériaux selon les revendications 1 à 6, caractérisés en ce qu'ils sont réalisés sous la forme d'une matière composite cohésive, plastique ou rigide à la température ambiante et aussi de préférence à la température du corps, matière qui contient le composant de la résine en phase fermée, dans laquelle les matières céramiques se présentent dispersées en une répartition de préférence homogène.

8. Matériaux selon les revendications 1 à 6, caractérisés en ce qu'ils sont réalisés en un matériau composite dispersé, dont les particules individuelles sont formées par des particules de céramique ou des agglomérats de particules, qui sont enveloppés par le composant de résine et sont éventuellement imprégnés par celui-ci au moins partiellement.

9. Matériaux selon les revendications 1 à 8, caractérisés en ce qu'ils contiennent des composants de résine d'un poids moléculaire moyen dans la gamme de 200 à 10 000 g/mole et en particulier de 300 à 5 000 g/mole.

10. Matériaux selon les revendications 1 à 9, caractérisés en ce qu'ils sont présents sous forme d'une masse cohésive, plastique à chaud et contiennent les matières céramiques, exclusivement sous forme pulvérulente avec des teneurs de cette poudre supérieure à environ 30 % en poids, de préférence dans la gamme de 40 à 65 % en poids, la taille des particules préférées de la poudre se situant en dessous de $100 \times 10^{-6}$ m.

11. Matériaux selon les revendications 1 à 9, caractérisés en ce qu'ils contiennent comme masse cohérente les matières céramiques sous forme de granulés - diamètre préférentiel des particules de granulés de 0,1 à 2 mm, en particulier de 0,2 à 1 mm, la teneur en granulés se situant de préférence dans la gamme de 20 à 60 % en poids et en particulier dans la gamme de 25 à 50 % en poids.

12. Matériaux selon les revendications 1 à 11, caractérisés en ce que les matières céramiques sont présentes dans une répartition dimensionnelle au moins bimodale, en particulier en un mélange de poudre et de granulés, dans lequel préférentiellement la proportion de poudre est constituée de 5 à 30 % en poids et la proportion de granulés de 15 à 50 % en poids, le pourcentage en poids étant rapporté à la matière totale.

13. Matériaux selon les revendications 1 à 12, caractérisés en ce que comme composant de résine sont présents des polymères de l'acide glycolique et/ou de l'acide lactique, qui ne contiennent pas de restes de catalyseurs étrangers de la préparation.